Europäisches Patentamt

European Patent Office

Office européen des brevets

(19)

(11) Publication number: **0 101 622**
**B1**

(12)

## EUROPEAN PATENT SPECIFICATION

(45) Date of publication of patent specification: 27.07.88

(21) Application number: 83200975.7

(22) Date of filing: 29.06.83

(51) Int. Cl.⁴: **B 01 J 23/80,** B 01 J 37/18,
C 07 C 29/15, C 07 C 31/04

(54) Modified copper, and zinc-containing catalyst and process for producing methanol using said catalyst.

(30) Priority: 19.07.82 US 399542
27.04.83 US 487081

(43) Date of publication of application:
29.02.84 Bulletin 84/09

(45) Publication of the grant of the patent:
27.07.88 Bulletin 88/30

(84) Designated Contracting States:
BE DE FR GB IT NL

(56) References cited:
EP-A-0 034 338
FR-A-2 441 420
US-A-3 758 417
US-A-3 961 037
US-A-4 279 781
US-A-4 298 354

(73) Proprietor: SHELL INTERNATIONALE
RESEARCH MAATSCHAPPIJ B.V.
Carel van Bylandtlaan 30
NL-2596 HR Den Haag (NL)

(72) Inventor: Ryan, Robert Charles
4914 Gleneagles
Houston Texas 77084 (US)
Inventor: Slaugh, Lynn Henry
12518 Texas Army Trail
Cypress Texas 77429 (US)

(74) Representative: Aalbers, Onno et al
P.O. Box 302
NL-2501 CH The Hague (NL)

Courier Press, Leamington Spa, England.

# 0 101 622

**Description**

This invention relates to a modified catalyst, comprising copper, zinc and a metal selected from the group consisting of ytrrium, a lanthanide element, an actinide element and mixtures thereof, which catalyst is particularly useful for converting carbon monoxide and hydrogen (syngas) to methanol.

There are a number of prior disclosures on copper and/or zinc-containing catalysts which are more or less related to the presently proposed one, and some of these prior disclosures also mention the use of converting syngas into methanol. Possibly most relevant is U.S. Patent Specification 3,758,417 disclosing a copper, zinc, and didymium oxide-containing catalyst suitable for converting carbon oxides and hydrogen to methanol. This catalyst is prepared by co-precipitation of nitrates at a temperature of about 85°C—90°C. No particular pH is indicated as critical.

The present invention relates to a modified catalyst composition having much higher activity, particularly with respect to the production of methanol as indicated above.

Accordingly, the present invention provides a catalyst composition which comprises copper, zinc, and a rare earth metal modifier selected from the group consisting of yttrium, a lanthanide element, an actinide element and mixtures thereof wherein the amount of said modifier is from 1 to 25 weight percent based on total catalyst composition, calculated as metal and wherein the relative proportions of copper to zinc are from 1:10 to 20:1, calculated as weight percent metal, said catalyst composition being prepared by a process comprising co-mixing an aqueous solution of copper, zinc and modifier salts with an aqueous solution of an alkali metal carbonate, thereby producing a precipitate, washing, drying, and calcining the obtained precipitate at a temperature ranging from 200°C to 400°C and activating in a hydrogen-containing atmosphere at a temperature ranging from 175°C to 400°C, characterized in that an aqueous solution of copper, zinc and modifier salts and an aqueous solution of an alkali metal carbonate are simultaneously co-mixed at a temperature of between 45°C and 85°C while maintaining a pH between 5.5 and 7.5.

Such catalyst compositions shown an activity which is substantially enhanced over catalysts known from the prior art, such as those taught by U.S. Patent Specification 3,758,417.

In the above a lanthanide element is one of the Lanthanide series comprising those elements with atomic numbers ranging from 57 (lanthanum) through 71 (luteium), whilst an actinide element is one of the Actinide series comprising those elements ranging from atomic number 89 up through atomic number of greater than 100. The higher numbers, because of scarcity, radio-activity and short lifetimes, do not really make satisfactory catalyst modifiers. Of interest in the Actinide series are those elements ranging from atomic number 89 up to 92 particularly those ranging from 90 to 92, particularly thorium and uranium and most preferably thorium. Mixtures of promoter elements may be suitably utilized. Particularly with regard to the Lanthanide (rare earth) series, mixed metals are readily available commercially. A suitable example of a mixed metal available commercially is the so called "didymium" metal. The composition of didymium is described in U.S. Patent Specification 3,758,417, referred to above. Another commercially available preparation of rare earths is that known as "misch-metal". Other mixtures of rare earths are also available.

The precise amount of modifier is not critical. In general, amounts of catalyst modifier of from 1 to 25% by weight, based on total catalyst composition and calculated as metal, are satisfactory, with amounts of catalyst modifier of from 4% to 15% by weight, on the same basis, being preferred.

The relative proportions of copper to zinc to be employed in the catalyst composition can be varied. In general, ratios from 1:10 to 20:1, calculated as weight percent metal, are satisfactory with ratios of from 1:1 to 10:1, calculated on the same basis, being preferred.

Preferred catalyst compositions are those, in which the modifier is selected from the group consisting of yttrium, a lanthandide element, thorium ane mixtures thereof. In other preferred compositions the modifier is praseodymium, neodymium, samarium, cerium, yttrium, lanthanum or thorium.

The initial form in which the copper, zinc and modifier are employed is preferably the oxide, although compounds which are readily converted to the oxide, e.g., the corresponding meal carbonates are also suitably initially employed as these are converted to the oxide, e.g. as during pretreatment subsequent to the formation of the initially prepared catalyst composition. Pretreatment of the catalyst in hydrogen and operation of the catalyst in the reaction environment will cause at least a partial reduction of some of the metals to lower oxidation states, and it is intended that catalysts with these reduced states will fall with the scope of this invention.

The compositions of the present invention are prepared in a very specific fashion in order to provide enhanced activity. Generally, the composition is prepared by co-precipitating the metals from a solution of their salts in the form of one or more compounds readily convertible to the oxides. The salts are preferably nitrates or are converted to nitrites by use of nitric acid to facilitate solution of non-nitrate salts. The precipitation is effected utilizing an aqeuous solution of an alkali metal carbonate which is co-mixed with the solution of metal salts at a given pH and a given temperature. In the preferred embodiment of the present invention, the copper, zinc and modifier nitrates are dissolved in water. A small amount of nitric acid is utilized to facilitate solution of non-nitrate compounds such as carbonates and oxides. A separate solution is prepared by dissolving an alkali metal carbonate in water, preferably potassium or sodium carbonate. The two solutions are then heated to the desired mixing temperature and simultaneously metered into a stirred precipitating container at individual rates such that a pH ranging from 5.5 to 7.5, preferably from 6.0 to 7.0, more preferably from 6.3 to 6.7, is maintained in the precipitating container. The

2

desired precipitating temperature ranges from 45°C to 85°C, preferably from 50°C to 80°C, more preferbaly from 50°C to 70C. The precipitate thus obtained is a mixture of carbonates, basic carbonates and hydroxides. It is then collected, washed substantially free of electrolytes, then dried and calcined at a temperature, as already mentioned, of from 200°C to 400°C, a temperature fo 250°C to 300°C being preferred. The drying is carried out at a temperature sufficient to remove the water. This step is conveniently combined with a calcination step by a suitable programming of the temperature from room temperature, slowly through the drying temperature, and then up to calcination temperature., The calcined material is shaped by, for example, pelleting under pressure using graphite as a lubricant. The oxide mixture is pretreated in a hydrogen-containing atmosphere prior to use as a catalyst to bring it to its most active state. Pretreatment is accomplished by contacting the catalyst with a stream of hydrogen, or hydrogen mixed with an inert gas or diluent at a temperature ranging from 175°C to 400°C. Suitable diluent gases for the activating gas mixture comprise nitrogen, or oxides of carbon.

In some applications it may be preferable to employ the catalyst on an inert support, e.g. silica and/or alumina, but in most modifications it is preferred to employ the catalyst unsupported.

In line with the above, the invention does not encompass a process for preparing the presently proposed catalyst composition, and — in consideration of the technical progress obtained thereby — a process for producing methanol from a mixture of hydrogen and carbon monoxide (syngas) in the presence of said catalyst composition, as well as methanol produced by the latter process. Broadly speaking, the two last-mentioned aspects will be the subject of the text following hereinafter.

As mentioned above, an improved process for producing methanol is obtained by contacting the catalyst of the present invention with hydrogen and carbon monoxide or mixtures of carbon oxides (syngas). Molar ratios of hydrogen to carbon oxides may range from 0.5:1 to 20:1, preferably from 2:1 to 10:1. Preferably a molar ratio of hydrogen to carbon monoxide at 2:1 or higher is preferred. Carbon dioxide may be present in the reaction mixture in amounts up to 50% by weight. Reaction temperatures range from 200 to 325°C with pressures ranging from atmospheric pressure to 100,000 kPa. Gaseous hourly space velocities may range from 5 to 25,000 $h^{-1}$.

The catalyst composition may be employed in batchwise operations or in a continuous manner as by passing the reactants through a tubular reactor containing the catalyst the maintained at reaction temperature.

After reaction the product mixture is separated and the methanol is recovered by conventional methods, e.g. selective condensation or selective adsorption.

The present invention will be illustrated by the following embodiments which are provided for illustration only and are not to be construed as limiting the invention.

Example 1

120.7 g of $Cu(No_3)_2.3H_2O$ and 60 g of $Zn(NO_3)_2$ are added to 500 ml of water. 4.1 g of a mixture of rare earth carbonates (Moly Corp., 40% La, 12% Nd, 4% Pr, 2½% Sm) are added to 50 ml of water to which 3.5 ml of nitric acid is slowly added to facilitate the solution of the carbonate compounds. This latter solution was slowly added to the copper/zinc solution followed by sufficient water to make 1 l of solution. A 1 molar solution of sodium carbonate was prepared. Both solutions were heated to 85°C, and the two solution were individually metered to a stirred 4 l beaker at a temperature of 65°C to give a slurry with a pH of 6.5. After the copper-zinc-rare earth metal solution was added to the beaker, the metering of the sodium carbonate solution was terminated, the precipitate was then filtered and washed with 20 l of water. The precipitate was then dried in a vacuum oven for 16 h. The dried precipitate was charged to a large glass tube reactor, heated slowly to 265°C under a stream of upflow air for 4 h to calcine the material. The composition was then activated in hydrogen for 1000 ml/min at a temperature up to 225°C. The resulting catalyst composition is noted as Example 1 in Table 1 which also demonstrates its use in the preparation of methanol from syngas.

Comparative Example A

A comparative example was prepared according to the teachings of U.S. Patent Specification 3,758,417. A first solution was prepared by dissolving 95 g of sodium carbonate in 1 l of water. A second solution was prepared by dissolving 120.7 g of $Cu(NO_3)_2$, 60 g of $Zn(NO_3)_2$, 4.1 g of the mixture of rare earth carbonates described in Example 1 in 50 ml of water and 3.5 ml of nitric acid. Water was then added to the second solution to bring it up to 1 l. Both solutions were then heated to 90°C and the second solution was quickly mixed with the first solution together with rapid stirring in 15 min. During the precipitation which occurred the pH ranged from 8.6 to 7.5 at the termination of precipitation. The precipitate was then washed 16 times with 1 l of water, dried in a vacuum oven at 120°C at 3.4 kPa for 16 h. The precipitate was then charged to a large glass tube reactor and slowly heated to 265°C under a stream of upflow air for 16 h to calcine the material. The calcined material was then activated in hydrogen at 1000 ml/min at a temperature of 225°C.

Comparative Example B

Another comparative example was prepared similar to that of Example A above except that the order of addition of the solutions was reversed, i.e. the sodium carbonate solution was added to the metal nitrate solution. In this example the calcination was carried out with a 2:1 (by volume) nitrogen/hydrogen mixture at 1200 ml/min.

### Comparative Example C

This example was prepared in the same fashion as Example B except that the calcination was carried out using 600 ml/min.

### Comparative Example D

This example was prepared in a fashion similar to Example B above, except that the calcination was carried out with hydrogen at 400 ml/min.

### Comparative Example E

This comparative example involved a two-stage precipitation process. A solution of rare earth and zinc nitrates were precipitated at 70°C with sodium carbonate, and then a solution of copper and zinc nitrate was added to this slurry of precipitates, and the resultant mixture was further precipitated at 70°C with additional sodium carbonate.

### Comparative Example F

This comparative example was prepared in a fashion similar to Example E except that the second precipitation was carried out at 85°C.

### Example 2

The catalysts prepared above were tested for their activity for the conversion of syngas to methanol. To perform this comparison a catalyst composition was loaded into a tube reactor. A mixture of hydrogen and carbon monoxide in a molar ratio of 2:1 was introduced into the reactor at a temperature of 300°C and at the flow rates listed in Table 1. Analyses of the liquid product are also shown in Table 1. As can be seen from this table, the catalyst of this invention gives a much higher liquid product yield than catalyst prepared, for example, in accordance with the teachings of U.S. Patent Specification 3,758,417 (note col., 3, lines 33—36, and Example A in this specification).

### Examples 3—11

Additional catalysts were prepared similar to those methods illustrated above in Example 1 and Comparative Example A. These were tested at a temperature of 240°C for their activity for the conversion of syngas to methanol in the manner described above. Results are shown in Table 2.

4

TABLE 1

| Example | Catalyst (%w) | Surface area (m²/g) | Temp (°C) Max Yield | GHSV (10⁻³) | Liquid Product Yield (kg/l-cat/h) | Liquid Product (%w) | | | |
|---|---|---|---|---|---|---|---|---|---|
| | | | | | | MeOH | EtOH | Higher organics | H₂O |
| 1 | Cu 38 Zn 14 RE 5 | 45 | 300 | 13.8 | 1.25 | 95.6 | 1.2 | 2.1 | 1.1 |
| A | Cu 40 Zn 15 RE 5 | 42 | 300 | 14.4 | 1.07 | 97.0 | 1.2 | 0.9 | 0.9 |
| B | Cu 50 Zn 19 RE 4 | 8 | 300 | 8.9 | 0.50 | 96.2 | 1.5 | 1.5 | 0.8 |
| C | Cu 50 Zn 19 RE 4 | 8 | 300 | 7.4 | 0.40 | 91.0 | 2.1 | 5.5 | 1.4 |
| D | Cu 50 Zn 19 RE 4 | 8 | 300 | 7.0 | 0.33 | 89.8 | 1.9 | 6.7 | 1.6 |
| E | Cu 35 Zn 16 RE 14 | 11 | 325 | 8.9 | 0.26 | 88.5 | 1.4 | 8.7 | 1.4 |
| F | Cu 44 Zn 16 RE 7 | 21 | 300 | 13.9 | 0.88 | 98.9 | 0.4 | 0.1 | 0.6 |

TABLE 2

COPPER – ZINC – MODIFIER METHANOL CATALYSTS

Syngas Feed: 65.9% $H_2$, 19.2% CO, 14.9% $CO_2$

GHSV = 15,000

Pressure = 5250 kPa

Temperature = 238 – 240°C

| | Catalyst Composition | | | Surface | Catalyst Preparation Procedure | | | | | |
|---|---|---|---|---|---|---|---|---|---|---|
| Examples | %w Cu | %w Zn | %w Modifier | area $m^2/g$ | Method [a] | Base reagent | pH | Temp °C | Yield of $CH_3OH$ g $CH_3OH$/ml Cat./h |
| G | 53.4 | 26.6 | 0 | 49 | A | $Na_2CO_3$ | 6.5 | 60 | 1.2 |
| 2 | 59.0 | 17.6 | 3.6 La | 76 | A | $Na_2CO_3$ | 6.5 | 60 | 1.60 |
| H | 60.8 | 17.8 | 1.5 La | 52 | B | $Na_2CO_3$ | ? | 85 | 0.47 |
| I | 52.3 | 16.9 | 11.6 La | 44 | B | $Na_2CO_3$ | ? | 85 | 0.2 |
| 3 | 61.7 | 13.9 | 4.8 Th | – | A | $Na_2CO_3$ | 6.5 | 60 | 1.8 |
| J | 63.3 | 13.1 | 4.0 Th | 36 | B | $Na_2CO_3$ | ? | 85 | 0.13 |
| 4 | 56.8 | 20.2 | 2.9 Y | – | A | $Na_2CO_3$ | 6.5 | 60 | 1.77 |
| 5 | 55.1 | 18.2 | 1.7 Y | – | A | $Na_2CO_3$ | 6.5 | 60 | 2.0 |
| 6 | 59.6 | 17.8 | 2.7 Ce | – | A | $Na_2CO_3$ | 6.5 | 60 | 2.43 |
| 7 | 57.5 | 19.0 | 3.8 Sm | – | A | $Na_2CO_3$ | 6.5 | 60 | 2.16 |
| 8 | 58.2 | 19.0 | 3.0 Nd | – | A | $Na_2CO_3$ | 6.5 | 60 | 2.3 |

TABLE 2 (Cont'd)

COPPER – ZINC – MODIFIER METHANOL CATALYSTS

Syngas Feed: 65.9% $H_2$, 19.2% CO, 14.9% $CO_2$

GHSV = 15,000

Pressure = 5250 kPa

Temperature = 238 – 240°C

| Examples | Catalyst Composition | | | Surface area $m^2/g$ | Catalyst Preparation Procedure | | | | Yield of $CH_3OH$ g $CH_3OH$/ml Cat./h |
| | %w Cu | %w Zn | %w Modifier | | Method [a] | Base reagent | pH | Temp °C | |
|---|---|---|---|---|---|---|---|---|---|
| 9 | 59.9 | 17.7 | 2.5 Pr | – | A | $Na_2CO_3$ | 6.5 | 60 | 2.16 |
| 10 | 60.8 | 17.1 | 2.5 Sc | – | A | $Na_2CO_3$ | 6.5 | 60 | 0.43 |
| 11 | 56.8 | 20.0 | 3.4 [b] | – | A | $Na_2CO_3$ | 6.5 | 60 | 2.0 |

[a] "A" method: A metal nitrates solution and a basic solution were simultaneously added to a stirred vat of water at a controlled pH and temperature (Similar to Example 1).

"B" method: A metal nitrates solution at 85°C was rapidly added to a stirred, hot (85°C) $Na_2CO_3$ solution. The pH was not controlled carefully (Similar to Example A).

[b] Used for catalyst preparation was "Mischmetal" rare earth reduced metal mixture dissolved in nitric acid: 0.3% Y, 51.6% Ce, 5.6% Pr; 18.7% Nd and 23.7% La.

**Claims**

1. A catalyst composition which comprises copper, zinc, and rare earth metal modifier selected from the group consisting of yttrium, a lanthanide element, an actinide element and mixtures thereof wherein the amount of said modifier is from 1 to 25 weight percent based on total catalyst composition, calculated as metal and wherein the relative proportions of copper to zinc are from 1:10 to 20:1, calculated as weight percent metal, said catalyst composition being prepared by a process comprising co-mixing an aqueous solution of copper, zinc and modifier salts with an aqueous solution of an alkali metal carbonate, thereby producing a precipitate, washing, drying, and calcining the obtained precipitate at a temperature ranging from 200°C to 400°C and activating in a hydrogen-containing atmosphere at a temperature ranging from 175°C to 400°C, characterized in that an aqueous solution of copper, zinc and modifier salts and an aqueous solution of an alkali metal carbonate are simultaneously co-mixed at a temperature of between 45°C and 85°C while maintaining a pH between 5.5 and 7.5.

2. The composition as claimed in claim 1, wherein the modifier is present in the composition in an amount of from 4 to 15 weight percent based on total catalyst composition, calculated as metal.

3. The composition as claimed in claim 1 or 2, wherein the copper and zinc are present in the composition in a weight ratio ranging from 1:1 to 10:1.

4. The composition as claimed in any one of claims 1—3, wherein the modifier is selected from the group consisting of yttrium, a lanthanide element, thorium and mixtures thereof.

5. The composition as claimed in any one of claims 1—3, wherein the modifier is praseodymium, neodymium, samarium, cerium, yttrium, lanthanium or thorium.

6. The composition as claimed in any one of claims 1—5, wherein the simultaneous co-mixing takes place at a pH ranging from 6.0 to 7.0.

7. The composition as claimed in claim 6, wherein the pH is ranging from 6.3 to 6.7.

8. The composition as claimed in claim 1—7, wherein the simultaneous co-mixing takes place at a temperature of betwen 50°C and 80°C.

9. The composition as claimed in claim 8, wherein the temperature is between 50°C and 70°C.

10. The composition as claimed in any one of claims 1—9, wherein the copper, zinc and modifier are in the form of nitrates.

11. The composition as claimed in any one of claims 1—9, wherein the copper, zinc and modifier are at least partly in the form of oxides.

12. A process for producing methanol from a mixture of hydrogen and carbon monoxide in the presence of a catalyst composition as claimed in any one of claims 1—11.

**Patentansprüche**

1. Katalysator aus Kupfer, Zink und Edelmetall- Modifizier- mittel ausgewählt aus der Gruppe, bestehend aus Yttrium, einem Lantaniden-Element, einem Aktiniden-Elemet und deren Gemischen, wobei die Menge des Modifiziermittels, berechnet als Metall, bezogen auf den gesamten Katalysator 1 bis 25 Gew.-% beträgt und wobei die relativen Anteile von Kupfer zu Zink 1:10 bis 20:1, berechnet als Gewichtsprozent Metall, betragen, der Katalysator hergestellt worden ist nach einem Verfahren, umfassend das gemeinsame Vermischen einer wäßrigen Lösung von Kupfer, Zink und Modifiziermittelsalzen mit einer wäßrigen Lösung eines Alkalicarbonats, unter Bildung eines Niederschlags, Waschen, Trocknen und Calcinieren des erhaltenen Niederschlags bei einer Temperatur im Bereich von 200 bis 400°C und Aktivieren in einer wasserstoffhaltigen Atmosphäre bei einer Temperatur von 175 bis 400°C, dadurch gekennzeichnet, daß eine wäßrige Lösung von Kupfer, Zink und Modifiziermittelsalzen und eine wäßrige Lösung eines Alkalicarbonats gleichzeitig zwischen 45°C und 85°C vermischt werden, während der pH-Wert zwischen 5,5 und 7,5 gehalten wird.

2. Katalysator nach Anspruch 1, wobei das Modifiziermittel in dem Katalysator in einer Menge von 4 bis 15 Gew.-%, bezogen auf den gesamten Katalysator, berechnet als Metall, vorliegt.

3. Katalysator nach Anspruch 1 oder 2, wobei das Kupfer und Zink in dem Katalysator in einem Gewichtsvershältnis von 1:1 bis 10:1 vorliegen.

4. Katalysator nach einem der Ansprüche 1 bis 3, wobei das Modifiziermittel ausgewählt ist aus der Gruppe, bestehend aus Yttrium, einem Lanthaniden-Element, Thorium und deren Gemischen.

5. Katalysator nach einem der Ansprüche 1 bis 3, wobei das Modifiziermittel Praseodym, Neodym, Samarium, Cer, Yttrium, Lanthan oder Thorium ist.

6. Katalysator nach einem Ansprüche 1 bis 5, wobei das gleichzeitige Vermischen bei einem pH-Wert im Bereich von 6,0 bis 7,0 erfolgt.

7. Katalysator nach Anspruch 6, wobei der pH-Wert im Bereich von 6,3 bis 6,7 liegt.

8. Katalysator nach einem der Ansprüche 1 bis 7, wobei das gleichzeitige Vermischen bei einer Temperatur zwischen 50°C und 80°C stattfindet.

9. Katalysator nach Anspruch 8, wobei die Temperatur zwischen 50 und 70°C liegt.

10. Katalysator nach einem Ansprüche 1 bis 9, wobei Kupfer, Zink und Modifiziermittel in Form von Nitraten vorliegen.

11. Katalysator nach einem Ansprüche 1 bis 9, wobei Kupfer, Zink un Modifiziermittl zumindest teilweise in Form von Oxiden vorliegen.

12. Verfahren zur Herstellung von Methanol aus einem Gemisch von Wasserstoff und Kohlenmonoxid in Gegenwart eines Katalysators nach einem der Ansprüche 1 bis 11.

**Revendications**

1. Une composition catalytique qui comprend du cuivre, du zinc et un modificateur métal de terre rare choisi dans le groupe constitué par l'yttrium, un élément des lanthanides, un élément des actinides et leurs mélanges, où la quantité du modificateur est comprise entre 1 et 25% en poids par rapport à la composition catalytique totale, en calculant en métaux, et où les proportions relatives du cuivre au zinc sont comprises entre 1:10 et 20:1, en calculant en pourcentage en poids de métaux, cette composition catalytique étant préparée par un procédé comprenant le mélange mutuel d'une solution aqueuse de sels de cuivre, de zinc et de modificateur avec une solution aqueuse d'un carbonate de métal alcalin, de manière à produire un précipité, le lavage, le séchage et la calcination du précipité obtenu à une température comprise entre 200°C et 400°C et l'activation dans une atmosphère contenant de l'hydrogène à une température comprise entre 175°C et 400°C, caractérisée en ce qu'une solution aqueuse de sels de cuivre, de zinc et de modificateur et une solution aqueuse d'un carbonate de métal alcalin sont mélangées ensemble simultanément à une température comprise entre 45°C et 85°C tandis qu'on maintient un pH compris entre 5,5 et 7,5.

2. La composition selon la revendication 1, dans laquelle le modificateur est présent dans la composition à raison de 4 à 15% en poids par rapport à la composition catalytique totale, en calculant en métaux.

3. La composition selon la revendication 1 ou 3 dans laquelle le cuivre et le zinc sont présents dans la composition dans un rapport en poids compris entre 1:1 et 10:1.

4. La composition selon l'une quelconque des revendications 1—3, dans laquelle le modificateur est choisi dans le groupe constitué par l'yttrium, un élément des lanthanides, le thorium et leurs mélanges.

5. La composition selon l'une quelconque des revendications 1—5, dans laquelle le modificateur est du praséodyme, du néodyme, du samarium, du cérium, de l'yttrium, du lanthane ou du thorium.

6. La composition selon l'une quelconque des revendications 1—5, dans laquelle le mélange mutuel simultané a lieu à un pH compris entre 6,0 et 7,0.

7. La composition selon la revendication 16, dans laquelle le pH est compris 6,3 et 6,7.

8. La composition selon l'une quelconque des revendications 1—7, dans laquelle le mélange mutuel simultané a lieu à une temperature comprise entre 50°C et 80°C.

9. La composition selon la revendication 18, dans laquelle la température est comprise entre 50°C et 70°C.

10. La composition selon l'une quelconque des revendications 1—9, dans laquelle le cuivre, le zinc et le modificateur sont sous la forme de nitrates.

11. La composition selon l'une quelconque des revendications 1—9, dans laquelle le cuivre, le zinc et le modificateur sont au moins partiellement sous la forme d'oxydes.

12. Un procédé de production de méthanol à partir d'une mélange d'hydrogène et d'oxyde de carbone en présence d'une composition catalytique telle que revendiquée dans l'une quelconque des revendications 1—11.